# EUROPEAN PATENT SPECIFICATION

(11) **EP 2 839 784 B1**
(45) Date of publication and mention of the grant of the patent: **22.03.2017**
(21) Application number: 13854508.2
(22) Date of filing: 21.10.2013
(51) Int. Cl.: A61B 8/12, B06B 1/02

(54) **BIAS-VOLTAGE GENERATION DEVICE, AND ULTRASOUND DIAGNOSTIC SYSTEM**
VORRICHTUNG ZUR ERZEUGUNG VON VORSPANNUNG UND ULTRASCHALLDIAGNOSESYSTEM
DISPOSITIF DE GÉNÉRATION DE TENSION DE POLARISATION, ET SYSTÈME DE DIAGNOSTIC À ULTRASONS

(30) Priority: 16.11.2012 JP 2012252427
(43) Date of publication of application: 25.02.2015
(73) Proprietor: Olympus Corporation, Tokyo 192-8507 (JP)
(72) Inventor: KOMURO, Masahiko, Hachioji-shi, Tokyo 192-8507 (JP)
(74) Representative: Gunzelmann, Rainer
(86) International application number: PCT/JP2013/078429
(87) International publication number: WO 2014/077087

(56) References cited:
- JP-A- S6 222 638
- JP-A- 2003 010 177
- JP-A- 2003 339 708
- JP-A- 2007 097 760
- JP-A- 2007 125 225

## Description

### Technical Field

The present invention relates to a bias voltage generating apparatus for performing ultrasound observation using a capacitive transducer and an ultrasound diagnostic system.

### Background Art

Ultrasound diagnostic systems including an ultrasound probe, an insertion portion of which is inserted into a body cavity to transmit/receive ultrasound to/from an affected part or the like, have been widely used in recent years.

An ultrasound probe using a capacitive transducer has been adopted as an ultrasound probe for transmitting/receiving ultrasound, in addition to an ultrasound probe using a piezoelectric element. In a case of an ultrasound probe equipped with a piezoelectric element, transmission signal generating means and reception signal processing means are provided on an ultrasound observation apparatus side.

A case using a capacitive transducer requires superimposition of a DC bias voltage that is a high voltage on a transmission signal and a received signal, which is unnecessary in a case using a piezoelectric element. For the reason, an ultrasound observation apparatus used together with an ultrasound probe equipped with a capacitive transducer has a configuration for an ultrasound observation apparatus for a piezoelectric element and further incorporates a DC bias voltage generating circuit which generates a DC bias voltage.

In contrast, the conventional example in Japanese Patent Application Laid-Open Publication No. 2007-97760 discloses an ultrasound probe apparatus which has a capacitive transducer formed at a semiconductor substrate and DC bias voltage means provided at the semiconductor substrate for generating a DC bias voltage and further incorporates transmission signal generating means for generating a transmission signal (a drive signal), thus allowing size reduction of the apparatus.

In the conventional example, however, even if a connector is employed that is connectable to an ultrasound observation apparatus to be used (for ultrasound diagnosis) with an ultrasound probe equipped with a piezoelectric element connected thereto, the ultrasound probe apparatus cannot be used with the ultrasound observation apparatus. In other words, the conventional example requires an ultrasound observation apparatus dedicated for the ultrasound probe apparatus.

The conventional example thus has a demerit that it requires not only an existing ultrasound observation apparatus to be used together with an ultrasound probe equipped with a transducer including a piezoelectric element but a dedicated ultrasound observation apparatus.

This leads to a demand for apparatuses with wide applicability which, for example, can be utilized for ultrasound diagnosis even when an ultrasound observation apparatus for a piezoelectric element is used.

JP 2007 125225 A concerns an ultrasonic imaging apparatus that comprises a mainframe and an ultrasonic probe connected by a connecting cable. The connecting cable which is an optical fiber metal composite cable includes a metal wire and optical fibers. The main body is provided with an E/O element and an O/E element. The ultrasonic probe is provided with an O/E element and an E/O element. The ultrasonic probe is also provided with a mixed device chip including an optical communication module and a cMUT. The signal transmitting and receiving between the main body and the ultrasonic probe is performed by optical signals through the optical fibers.

The present invention was made in view of the above-mentioned points, and an objective of the present invention is to provide a bias voltage generating apparatus and an ultrasound diagnosis system capable of performing ultrasound diagnosis with an ultrasound probe equipped with a capacitive transducer even when an ultrasound observation apparatus for a piezoelectric element is used.

### Disclosure of Invention

### Means for Solving the Problem

A bias voltage generating apparatus according to one aspect of the present invention comprises the features of claim 1.

An ultrasound diagnostic system according to one aspect of the present invention includes the features of claim 9.

### Brief Description of the Drawings

Fig. 1 is a diagram showing an overall configuration of an ultrasound diagnostic system according to a first embodiment, not showing all elements of the present invention;
Fig. 2 is a diagram showing a configuration in which a bias voltage generating apparatus provided inside an ultrasound probe equipped with a capacitive transducer is connected to a bias voltage charger to perform charging;
Fig. 3 is a diagram showing a configuration in which the ultrasound probe equipped with the capacitive transducer is connected to an observation apparatus for a capacitive transducer to transmit/receive ultrasound;
Fig. 4 is a diagram showing a configuration in which the ultrasound probe equipped with the capacitive transducer is connected to an observation apparatus for an ultrasound probe equipped with a piezoelectric element to transmit/receive ultrasound;
Figs. 5 are timing charts for explaining action of the first embodiment;
Fig. 6 is a diagram showing an overall configuration of an ultrasound diagnostic system according to a first modification of the first embodiment;
Figs. 7 are timing charts for explaining action of the first modification;
Fig. 8 is a diagram showing a configuration of a bias voltage generating apparatus and the like according to a second modification of the first embodiment;
Fig. 9 is a diagram showing a configuration of a bias voltage generating apparatus and the like according to a third modification of the first embodiment;
Fig. 10 is a diagram showing a configuration of a main portion of a first ultrasound probe constituting a second embodiment, embodying the present invention;
Figs. 11 are timing charts for explaining action using Fig. 10;
Fig. 12 is a diagram showing a configuration of a main portion of an electronic scanning-based first ultrasound probe according to a modification of the second embodiment; and
Fig. 13 is a diagram showing a configuration of a main portion of a first ultrasound probe that is a modification of the first ultrasound probe in Fig. 10.

### Best Mode for Carrying Out the Invention

Embodiments, the second of which is an embodiment of the present invention will be described below with reference to the drawings.

### (First Embodiment)

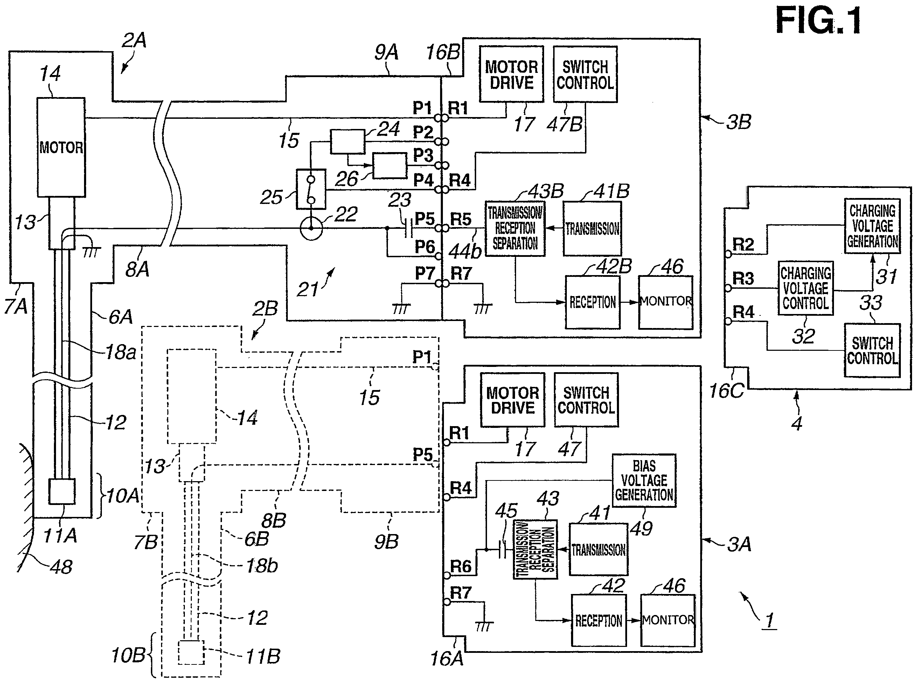

As shown in Fig. 1, an ultrasound diagnostic system 1 according to a first embodiment has a first ultrasound probe 2A which is based on mechanical scanning and is equipped with a capacitive transducer (abbreviated as a cMUT), a second ultrasound probe 2B which is based on mechanical scanning and is equipped with a transducer using a piezoelectric element, a first ultrasound observation apparatus (first observation apparatus) 3A to which the first ultrasound probe 2A is detachably connected and which produces an ultrasound image, a second ultrasound observation apparatus (second observation apparatus) 3B to which the first and second ultrasound probes 2A and 2B can be detachably connected and which produces an ultrasound image, and a bias voltage charger 4 which charges a power supply circuit 24 for bias voltage provided in the first ultrasound probe 2A. Note that Fig. 1 shows a state in which the first ultrasound probe 2A is connected to the second observation apparatus 3B.

The first and second ultrasound probes 2I (I = A or B) each include an elongated insertion portion 6I, a grasping portion (operation portion) 7I which is provided at a rear end (proximal end) of the insertion portion 6I, and a cable portion 8I which extends from the grasping portion 7I. A connector 9I is provided at an end portion of the cable portion 8I.

A cMUT 11 A is housed in a distal end portion 10A of the insertion portion 6A in the first ultrasound probe 2A. The cMUT 11A is coupled to a motor 14 in the grasping portion 7A via, for example, a hollow flexible shaft 12 and a slip ring 13.

The motor 14 is connected to one end of a signal line 15 in the cable portion 8A, and the other end of the signal line 15 is connected to a connector terminal (contact) P1 of the connector 9A. The signal line 15 is connected to a motor drive circuit 17 in the observation apparatus 3I via a connector receiver 16A or 16B to which the connector 9A is detachably connected.

A motor drive signal outputted from the motor drive circuit 17 is applied to the motor 14, which causes the motor 14 to be rotationally driven. With the rotation of the motor 14, the slip ring 13 and the flexible shaft 12 rotate. The rotation allows rotational driving of the cMUT 11 A attached to a distal end of the flexible shaft 12.

An electrode of the cMUT 11A is connected to one end of a signal line 18a which is inserted in the hollow flexible shaft 12. The signal line 18a inserted in the flexible shaft 12 is electrically continuous with the signal line 18a outside the slip ring 13 through a stator-side contact in contact with a rotor-side contact of the slip ring 13. The signal line 18a is connected to a connector terminal P5 via a bias voltage superimposing circuit 22 and a capacitor 23 constituting a bias voltage generating apparatus 21 which is arranged inside the connector 9A and is connected to a connector terminal P6 via the bias voltage superimposing circuit 22.

The bias voltage generating apparatus 21 includes the power supply circuit 24 for bias voltage that chargeably produces a bias voltage to be applied to the cMUT 11A. An input end of the power supply circuit 24 for bias voltage is connected to a connector terminal P2 for charging, and an output end of the power supply circuit 24 for bias voltage is connected to the bias voltage superimposing circuit 22 via a bias switch 25 as a switch for turning on/off application of the bias voltage.

In the power supply circuit 24 for bias voltage, charging level of a bias voltage is detected by a charging level detecting circuit 26. A signal line which outputs the detected charging level is connected to a connector terminal P3 functioning as a charging level terminal.

In the bias switch 25, a switch control terminal which controls turn-on/turn-off of two contacts of the bias switch 25 is connected to a connector terminal P4 via a signal line. The turn-on/turn-off of the two contacts of the bias switch 25 can be controlled by applying, for example, a binary switch control signal to the connector terminal P4 from an outside. That is, the connector terminal P4 forms a control terminal which controls turn-on/turn-off of application of a bias voltage in a bias voltage superimposing circuit 22 from the outside.

Note that a signal line connected to a ground-side electrode in the cMUT 11A is connected to a ground terminal of a connector terminal P7.

In the second ultrasound probe 2B, a transducer 11B formed from a piezoelectric element is housed in a distal end portion 10B of the insertion portion 6B, instead of the cMUT 11 A. The transducer 11 B is coupled to the motor 14 in the grasping portion 7B via, for example, the hollow flexible shaft 12 and the slip ring 13.

A signal line 18b which is connected to the transducer 11B and is inserted in the flexible shaft 12 is connected to the signal line 18b outside the slip ring 13 via the slip ring 13. The signal line 18b is connected to the connector terminal P5 of the connector 9B. Other components in the second ultrasound probe 2B are identical to the components in the first ultrasound probe 2A and are denoted by identical reference numerals in Fig. 1.

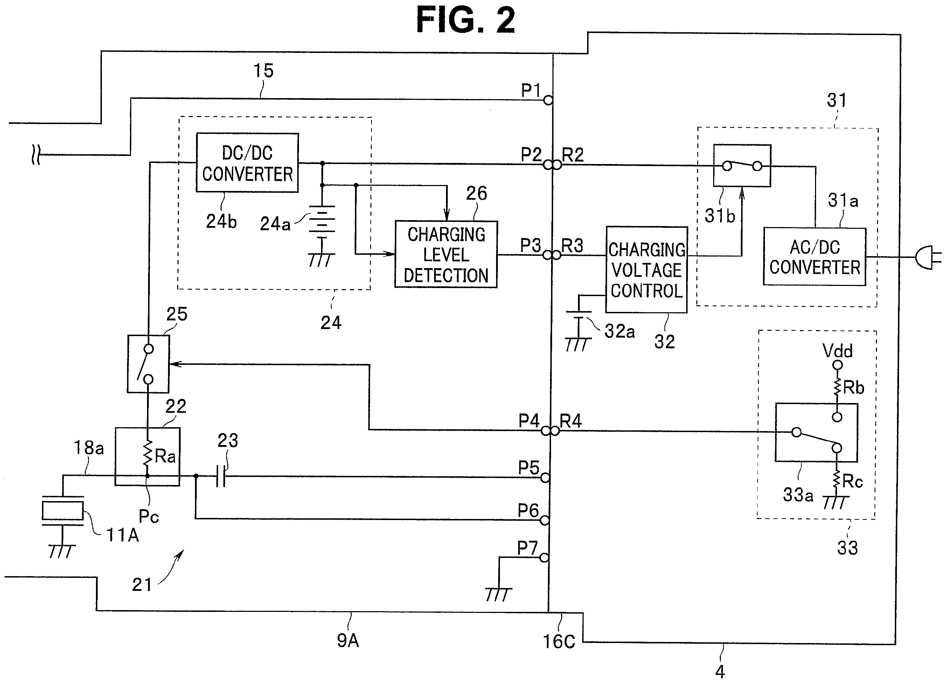

Fig. 2 shows a configuration of the bias voltage charger 4, to which the first ultrasound probe 2A is detachably connected.

The bias voltage charger 4 has a charging voltage generating circuit 31 which generates a charging voltage for charging the power supply circuit 24 for bias voltage, a charging voltage control circuit 32 which monitors the charging level detected by the charging level detecting circuit 26 and controls charging action of the charging voltage generating circuit 31, and a switch control circuit 33 which controls turn-on/turn-off of a bias switch.

The bias voltage charger 4 also has a connector receiver 16C having connector receiver terminals R2, R3, and R4, to which the connector terminals P2, P3, and P4 of the first ultrasound probe 2A are respectively connected.

More specifically, the connector receiver terminals R2, R3, and R4 are connected to an output end of the charging voltage generating circuit 31, an input end of the charging level detecting circuit 26, and an output end of the switch control circuit 33, respectively.

The charging voltage generating circuit 31 has an AC/DC converter 31 a which generates a DC voltage for charging a chargeable secondary battery 24a in the power supply circuit 24 for bias voltage from a commercial AC power supply and a switch 31 b which is provided at an output end of the AC/DC converter 31a. The charging voltage generating circuit 31 charges the secondary battery 24a by applying the DC voltage to the secondary battery 24a. Note that the secondary battery 24a is composed of a lithium battery, a lead battery, or the like which generates a DC voltage of, for example, about 5 V to about 10 V.

The power supply circuit 24 for bias voltage has the secondary battery 24a and a DC/DC converter 24b which raises a DC voltage of the secondary battery 24a to a bias voltage of 100 V or more.

A DC voltage of the secondary battery 24a is supplied to the charging level detecting circuit 26. The charging level detecting circuit 26 utilizes the DC voltage as a power supply for action, monitors the DC voltage of the secondary battery 24a, and detects the charging level. Note that the charging level detecting circuit 26 may detect the charging level from level of a bias voltage outputted from the DC/DC converter 24b.

The charging level detected by the charging level detecting circuit 26 is inputted to the charging voltage control circuit 32. The charging voltage control circuit 32 judges whether a fully charged state has been reached, by comparing a voltage corresponding to the charging level with a reference voltage 32a.

The charging voltage control circuit 32 performs control to turn off the switch 31b and end charging action when the voltage corresponding to the charging level reaches the reference voltage 32a. Note that lighting-up of an LED (not shown), extinguishment of the lighted LED, or the like may be performed on completion of charging to inform a user of a fully charged state. As will be described later, a charging level notifying circuit 72 may be connected to the charging level detecting circuit 26 to allow a user to be notified of the charging level.

The switch control circuit 33 is composed of a switch 33a which can be set for change-over to allow (through manual operation or the like) output of a switch control signal for control to turn off the bias switch 25 and resistors Rb and Rc which are connected to the switch 33a.

In the present embodiment, by setting the connector receiver terminal R4 to low level (L level) via the resistor Rc, the bias switch 25 is turned off via the connector terminal P4, to which the connector receiver terminal R4 is connected. Note that the bias switch 25 is a switch which is turned off when a voltage applied to a bias control terminal is, for example, at low level and is turned on when the voltage is at high level (H level).

The bias voltage superimposing circuit 22 as a bias voltage superimposing portion which superimposes a bias voltage on a transmission signal and a received signal is configured to superimpose a bias voltage on a junction Pc of the signal line 18a using, for example, a resistor Ra which is series-connected to the bias switch 25, as shown in Fig. 2.

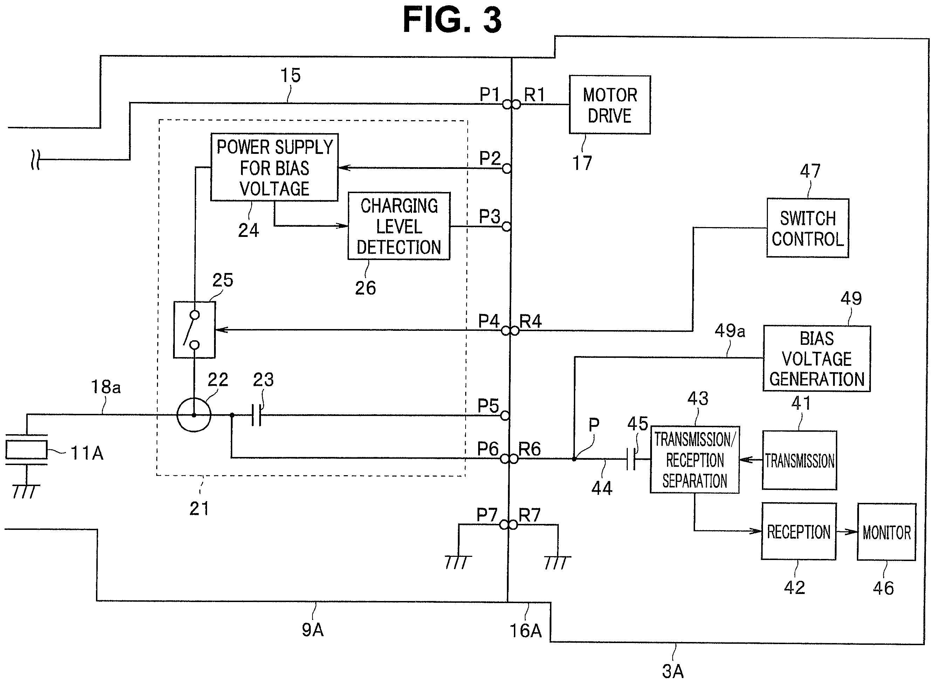

Fig. 3 shows a configuration of a main portion in a state in which the first ultrasound probe 2A is connected to the first observation apparatus 3A.

The first observation apparatus 3A has a transmission circuit 41 as a transmission signal generating portion which generates a transmission signal for transmission/reception of ultrasound to/from a subject using the cMUT 11A, a reception circuit 42 which performs processing on a received signal received by the cMUT 11A, and a bias voltage generating circuit 49 which generates a bias voltage to be superimposed on a transmission signal and a received signal. The bias voltage generated by the bias voltage generating circuit 49 is superimposed on a transmission signal or a received signal at a junction P with a signal line 44 for transmitting/receiving a transmission signal and a received signal via a signal line 49a. A function including generation of a bias voltage by the bias voltage generating circuit 49 and superimposition of the bias voltage at the junction P may be defined as a bias voltage circuit.

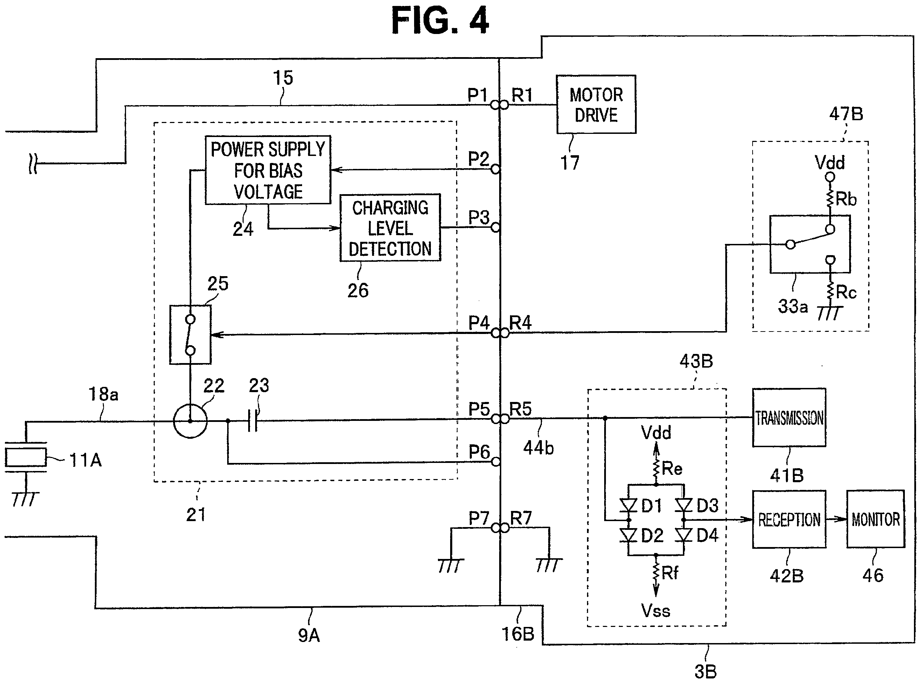

The first observation apparatus 3A has a capacitor 45 interposed in the signal line 44 for transmitting/receiving a transmission signal and a received signal to protect a bias voltage superimposed on the signal line 44 from being inputted to a transmission/reception separating circuit 43 as well as the transmission circuit 41 and the reception circuit 42. Note that the transmission/reception separating circuit 43 separates a transmission signal from a received signal using the common signal line 44. The transmission/reception separating circuit 43 has an identical configuration to a configuration of a transmission/reception separating circuit 43B shown in Fig. 4.

The signal line 44 is connected to the connector terminal P6 via a connector receiver terminal R6, and a connector receiver terminal R7 is connected to the connector terminal P7. A video signal of an ultrasound image which is produced by the reception circuit 42 is outputted to a monitor 46, and an ultrasound image of a subject which is produced through transmission and reception of ultrasound by the cMUT 11A is displayed on a display surface of the monitor 46.

A switch control circuit 47 which is provided at the first observation apparatus 3A is connected to the connector terminal P4 via the connector receiver terminal R4. The switch control circuit 47 may have an identical configuration to the configuration of the switch control circuit 33 shown in Fig. 2. That is, the switch control circuit 47 is set so as to output a switch control signal for turning off the bias switch 25, as shown in Fig. 3.

In the case, a bias voltage from the power supply circuit 24 for bias voltage in the connector 9A is not used to transmit and receive ultrasound.

In the case of the configuration in Fig. 3, the first observation apparatus 3A outputs a transmission signal together with a bias voltage superimposed on the transmission signal to a connector 9A side. The transmission signal with the bias voltage superimposed is applied to the cMUT 11A, and the cMUT 11A transmits ultrasound. Since the cMUT 11A is rotationally driven by the motor 14, the cMUT 11A transmits ultrasound to a subject 48 side, with which an outer circumferential face of the distal end portion 10A is in contact, as shown in Fig. 1.

The ultrasound transmitted to the subject 48 side is reflected at a portion changing in acoustic impedance. A portion of the reflected ultrasound is received by the cMUT 11A and is converted into an electric signal. In the case, since the predetermined bias voltage is applied to the cMUT 11A, the cMUT 11A can efficiently produce a converted received signal.

The received signal received by the cMUT 11A is inputted from the connector 9A into the first observation apparatus 3A while a bias voltage generated by the bias voltage generating circuit 49 in the first observation apparatus 3A is superimposed on the received signal. The received signal is inputted to the reception circuit 42 through the capacitor 45 and the transmission/reception separating circuit 43. A video signal is produced by the reception circuit 42. The video signal is inputted to the monitor 46, which results in display of an ultrasound image on the display surface of the monitor 46.

Fig. 4 shows a configuration of a main portion in a state in which the first ultrasound probe 2A is connected to the second observation apparatus 3B.

The second observation apparatus 3B has a transmission circuit 41B which generates a transmission signal for transmitting/receiving ultrasound to/from a subject using the transducer 11B that is a piezoelectric element if the ultrasound probe 2B is connected, a reception circuit 42B which performs processing on a received signal received by the transducer 11B, and the transmission/reception separating circuit 43B that separates a transmission signal from a received signal using a common signal line 44b in the second observation apparatus 3B. In the present embodiment, the transmission circuit 41B produces a transmission signal for the cMUT 11A, and the reception circuit 42B performs signal processing on a received signal received by the cMUT 11A, if the ultrasound probe 2A is connected, as shown in Fig. 4. The transmission/reception separating circuit 43B separates a transmission signal from a received signal.

As shown in Fig. 4, the transmission/reception separating circuit 43B is composed of a bridge of diodes D1 to D4 which are arranged in a signal line 44b' extending from a fork in the signal line 44b to an input end side of the reception circuit 42B, a resistor Re which connects anodes of the diodes D1 and D3 to a power supply end Vdd, and a resistor Rf which connects cathodes of the diodes D2 and D4 to a power supply end Vss. In a case of a weak signal (e.g., a signal of not more than 1 Vpp) such as a received signal, the bridge of diodes D1 to D4 is turned on, and the signal is inputted to the reception circuit 42B. In a case of an excessive signal (of, for example, about 100 Vpp) such as a transmission signal, the diode D1 or D2 is turned on, which causes the diode D3 or D4 to be turned off. The excessive signal is prevented from being applied to the reception circuit 42B.

Note that a diode having the property that a reverse recovery time period (recovery time period) is shorter than a period of a transmission signal may be used as each of the diodes D1 and D2 and that a diode having the property that a reverse recovery time period is longer than a period of a received signal may be used as each of the diodes D3 and D4, as described in Japanese Patent Application Laid-Open Publication No. 2011-229630.

Alternatively, as disclosed in Fig. 2 of Japanese Patent Application Laid-Open Publication No. 2010-201163, the bridge of diodes D1 to D4 and switches SW1 and SW2 for switching between a negative power supply and a positive power supply at a time of transmission and a time of reception, may be used. The bridge of diodes D1 to D4 may be made discontinuous at the time of transmission to protect a high-voltage transmission signal from being inputted to the reception circuit 42B. The bridge of diodes D1 to D4 may be made continuous at the time of reception to permit a received signal to be inputted to the reception circuit 42B.

In the present embodiment, as described above, the transmission circuit 41B and the reception circuit 42B can also be used as a transmission circuit which produces a transmission signal and a reception circuit which performs signal processing on a received signal for the cMUT 11A.

The connector receiver 16B of the second observation apparatus 3B has connector receiver terminals R1, R4, R5, and R7 which are connected to the connector terminals P1, P4, P5, and P7, respectively.

A video signal of an ultrasound image which is produced by the reception circuit 42B is outputted to the monitor 46. An ultrasound image of a subject which is produced through transmission and reception of ultrasound by the transducer 11B or the cMUT 11A is displayed on the display surface of the monitor 46.

A switch control circuit 47B which is provided at the second observation apparatus 3B is connected to the connector terminal P4 via the connector receiver terminal R4.

The switch control circuit 47B has an identical configuration to the configuration of the switch control circuit 33 shown in Fig. 2. The switch 33a is connected to the power supply end Vdd via the pull-up resistor Rb, and the switch control circuit 47B is set such that a switch control signal at H level is applied to the connector receiver terminal R4 and the connector terminal P4.

That is, the switch control circuit 47B controls the bias switch 25 to be turned on, as shown in Fig. 4.

In the case, a transmission signal from the transmission circuit 41 B is transmitted into the connector 9A via the transmission/reception separating circuit 43B, passes through the capacitor 23 in the connector 9A, and enters the bias voltage superimposing circuit 22. A bias voltage from the power supply circuit 24 for bias voltage provided in the connector 9A is superimposed on the transmission signal in the bias voltage superimposing circuit 22, and the transmission signal is applied to the cMUT 11A.

The bias voltage from the power supply circuit 24 for bias voltage provided in the connector 9A is also superimposed on a received signal which is obtained when ultrasound is received by the cMUT 11A. The received signal undergoes bias voltage cutting in the capacitor 23 and is then inputted into the second observation apparatus 3B. In the second observation apparatus 3B, the received signal is inputted to the reception circuit 42B via the transmission/reception separating circuit 43B. The reception circuit 42B performs signal processing on the received signal, produces a video signal, and outputs the video signal to the monitor 46.

The ultrasound diagnostic system 1 with the above-described configuration includes the first ultrasound probe 2A as an ultrasound probe that is equipped with the cMUT 11A as a capacitive transducer, the second observation apparatus 3B as an ultrasound observation apparatus that incorporates the transmission circuit 41B generating a transmission signal for causing the capacitive transducer to generate ultrasound, the reception circuit 42B performing signal processing on a received signal outputted from the capacitive transducer upon reception of ultrasound, and the transmission/reception separating circuit 43B separating the transmission signal from the received signal, and the bias voltage generating apparatus 21 that includes the power supply circuit 24 for bias voltage incorporated in the ultrasound probe outside the second observation apparatus 3B, including the chargeable secondary battery 24a for producing a bias signal to be applied to the capacitive transducer, and producing the bias voltage and the bias voltage superimposing circuit 22 as a bias voltage superimposing portion superimposing the bias voltage on the transmission signal outputted to outside the ultrasound observation apparatus and the received signal inputted into the ultrasound observation apparatus.

The bias voltage generating apparatus 21 provided in the first ultrasound probe 2A is the bias voltage generating apparatus 21 that is placed outside and used together with the second observation apparatus 3B as an ultrasound observation apparatus that incorporates the transmission circuit 41B generating a transmission signal for transmitting/receiving ultrasound to/from a subject using the cMUT 11A as a capacitive transducer and the reception circuit 42B performing processing on a received signal. The bias voltage generating apparatus 21 includes the power supply circuit 24 for bias voltage that includes the chargeable secondary battery 24a for producing a bias voltage to be applied to the capacitive transducer and produces the bias voltage and the bias voltage superimposing circuit 22 as a bias voltage superimposing portion that superimposes the bias voltage on the transmission signal outputted from the ultrasound observation apparatus and the received signal inputted to the ultrasound observation apparatus.

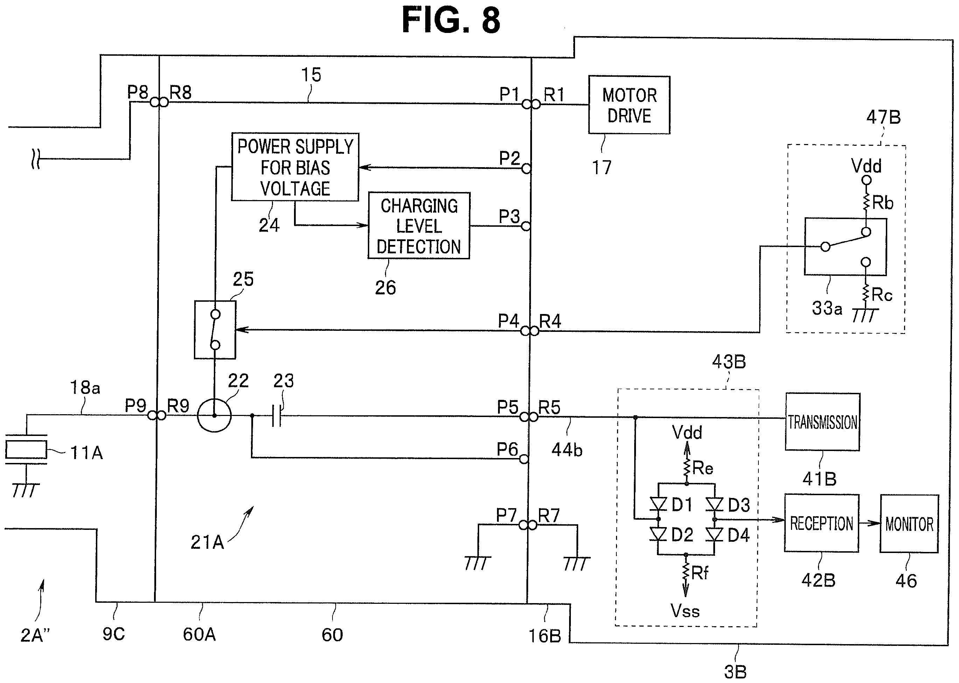

Note that although the bias voltage generating apparatus 21 is arranged inside the first ultrasound probe 2A equipped with the cMUT 11A in the above-described configuration, the bias voltage generating apparatus 21 is detachable from a first ultrasound probe 2A" equipped with the cMUT 11A outside the first ultrasound probe 2A" in the configuration in Fig. 8 to be described later.

Action of the present embodiment will be described. Before ultrasound diagnosis using the first ultrasound probe 2A, the power supply circuit 24 for bias voltage of the bias voltage generating apparatus 21 is connected to the bias voltage charger 4 to put the secondary battery 24a in a charged state, as shown in Fig. 2.

Since the bias voltage generating apparatus 21 is provided in the first ultrasound probe 2A in the present embodiment, the first ultrasound probe 2A is connected to the bias voltage charger 4 to charge the secondary battery 24a of the power supply circuit 24 for bias voltage at the bias voltage generating apparatus 21 provided in the first ultrasound probe 2A, as shown in Fig. 2.

It is possible to charge the power supply circuit 24 for bias voltage through the connection as shown in Fig. 2 and complete the charging upon notification through, e.g., lighting-up of an LED. After the completion of the charging, it is possible to transmit/receive ultrasound by connecting the first ultrasound probe 2A to the second observation apparatus 3B and perform ultrasound diagnosis. More specifically, the first ultrasound probe 2A is connected to the second observation apparatus 3B, as shown in Fig. 1 or 4.

In the case, a transmission signal from the transmission circuit 41B goes through the capacitor 23 in the connector 9A after passing through the transmission/reception separating circuit 43B. A bias voltage outputted from the power supply circuit 24 for bias voltage is superimposed on the transmission signal in the bias voltage superimposing circuit 22, and the transmission signal is applied to electrodes of the cMUT 11A.

In the cMUT 11A, application of the transmission signal with the bias voltage superimposed between electrodes on two sides facing across a cavity causes a membrane on one side facing the cavity to vibrate, and ultrasound is transmitted to the subject 48 side (see Fig. 1). The ultrasound is reflected on the subject 48 side, and a received signal received by the cMUT 11 A is inputted into the second observation apparatus 3B through the capacitor 23 while the bias voltage from the power supply circuit 24 for bias voltage is superimposed on the received signal.

In the second observation apparatus 3B, the received signal is inputted to the reception circuit 42B through the transmission/reception separating circuit 43B.
The reception circuit 42B performs signal processing on the received signal, produces a video signal, and outputs the video signal to the monitor 46. An ultrasound image is displayed on the display surface of the monitor 46. A surgeon observes the ultrasound image and diagnoses an affected part or the like.

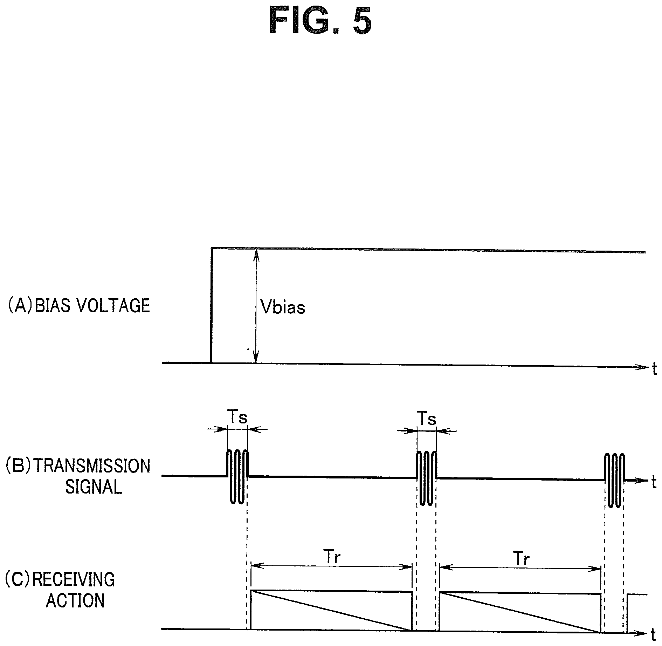

Figs. 5 show timing charts, and each horizontal axis indicates t. If the first ultrasound probe 2A is connected to the second observation apparatus 3B, a power supply switch (not shown) of the bias voltage generating apparatus 21 is turned on, and a power supply switch (not shown) of the second observation apparatus 3B is turned on, the power supply circuit 24 for bias voltage outputs a bias voltage Vbias as shown in Fig. 5(A), and the bias voltage Vbias is applied to the signal line 18a by the bias voltage superimposing circuit 22.

The transmission circuit 41B in the second observation apparatus 3B outputs a pulsed transmission signal as shown in Fig. 5(B) for a short transmission period Ts. The transmission signal with the bias voltage Vbias superimposed by the bias voltage superimposing circuit 22 is applied to the cMUT 11A, and ultrasound is transmitted.

When a reception period Tr after the transmission period Ts starts, as shown in Fig. 5(C), the reception circuit 42B becomes active and performs signal processing on a received signal received by the cMUT 11A.

Note that since the cMUT 11A is automatically rotationally driven in sync with rotation of the motor 14, a direction in which ultrasound is transmitted and received changes sequentially. By repeating transmission and reception as described above, the reception circuit 42B acquires a piece of image data for one frame in radial scanning. An ultrasound image which is produced from the piece of image data for one frame is displayed on the monitor 46.

Although the action of connecting the first ultrasound probe 2A to the second observation apparatus 3B for a piezoelectric element and transmitting/receiving ultrasound has been described, ultrasound can also be transmitted and received by connecting the first ultrasound probe 2A to the first observation apparatus 3A, as described with reference to Fig. 3.

Thus, according to the present embodiment, a bias voltage generating apparatus and an ultrasound diagnostic system which can be utilized for ultrasound diagnosis (or ultrasound observation) even if an ultrasound observation apparatus for a piezoelectric element (i.e., the second observation apparatus 3B) can be provided.

The above-described configuration relates to the mechanical scanning-based ultrasound diagnostic system 1. An electronic scanning-based ultrasound diagnostic system 1' can also be configured in the manner below.

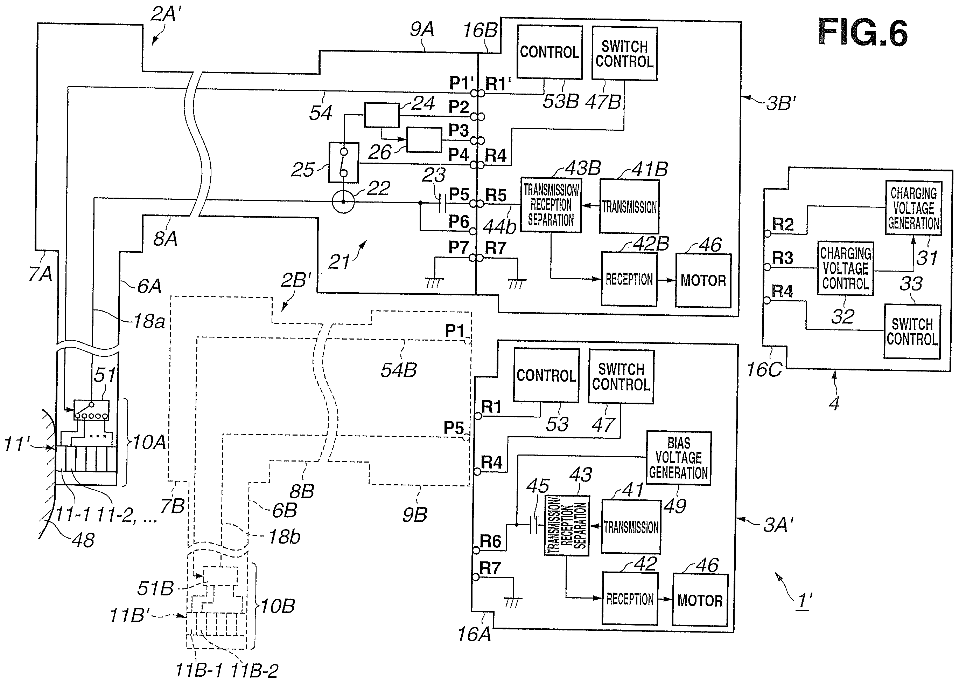

Fig. 6 shows a configuration of the electronic scanning-based ultrasound diagnostic system 1' according to a first modification of the first embodiment. The ultrasound diagnostic system 1' has a configuration obtained by partially changing, in the manner below, the ultrasound diagnostic system 1 in Fig. 1.

First and second ultrasound probes 2A' and 2B' according to the present modification are different from the first and second ultrasound probes 2A and 2B in Fig. 1 in that the first and second ultrasound probes 2A' and 2B' do not have the motor 14, the flexible shaft 12, and the slip ring 13. The first ultrasound probe 2A' has a cMUT array 11' having a plurality of cMUTs 11-1, 11-2,..., 11-m arranged along an inner circumferential face of the distal end portion 10A, instead of the one cMUT 11A in Fig. 1. The individual cMUTs 11-j (j = 1, 2,..., m) (electrodes of the cMUTs) are connected to a multiplexer 51 as transducer selecting means which is arranged in a vicinity of the cMUTs.

The multiplexer 51 is capable of sequentially selecting the cMUTs 11-j connected to the signal line 18a by a selection signal applied to the multiplexer 51.

A control circuit 53 in a first observation apparatus 3A' or a control circuit 53B in a second observation apparatus 3B' outputs a selection signal. The selection signal is applied to a transducer selection terminal of the multiplexer 51 through a connector receiver terminal R1' and a connector terminal P1' and then a signal line 54 which is inserted in the first ultrasound probe 2A'.

The control circuit 53 controls action of the transmission circuit 41 and the reception circuit 42 in the first observation apparatus 3A' in sync with a selection signal.

The second ultrasound probe 2B' has a cMUT array 11B' having a plurality of transducers 11B-1, 11B-2,..., 11B-m arranged along an inner circumferential face of the distal end portion 10B, instead of the transducer 11B composed of one piezoelectric element in Fig. 1. The individual transducers 11B-j (j = 1, 2,..., m) (electrodes of the transducers) are connected to a multiplexer 51B as transducer selecting means which is arranged in a vicinity of the transducers. The multiplexer 51B is capable of sequentially selecting the transducers 11B-j connected to the signal line 18b by a selection signal applied to the multiplexer 51B.

If the second ultrasound probe 2B' is connected to the second observation apparatus 3B', the control circuit 53B in the second observation apparatus 3B' outputs a selection signal. The selection signal is applied to the transducer selection terminal of the multiplexer 51B via a signal line 54B which is inserted in the second ultrasound probe 2B'.

The control circuit 53B controls action of the transmission circuit 41B and the reception circuit 42B in the second observation apparatus 3B' in sync with the selection signal.

If the first ultrasound probe 2A' is connected to the second observation apparatus 3B', the control circuit 53B outputs a selection signal in a same manner as in the case where the second ultrasound probe 2B' is connected. Note that the first observation apparatus 3A' and the second observation apparatus 3B' do not have the motor drive circuit 17, as shown in Fig. 6. Other components are the same as the components in the configuration shown in Fig. 1.

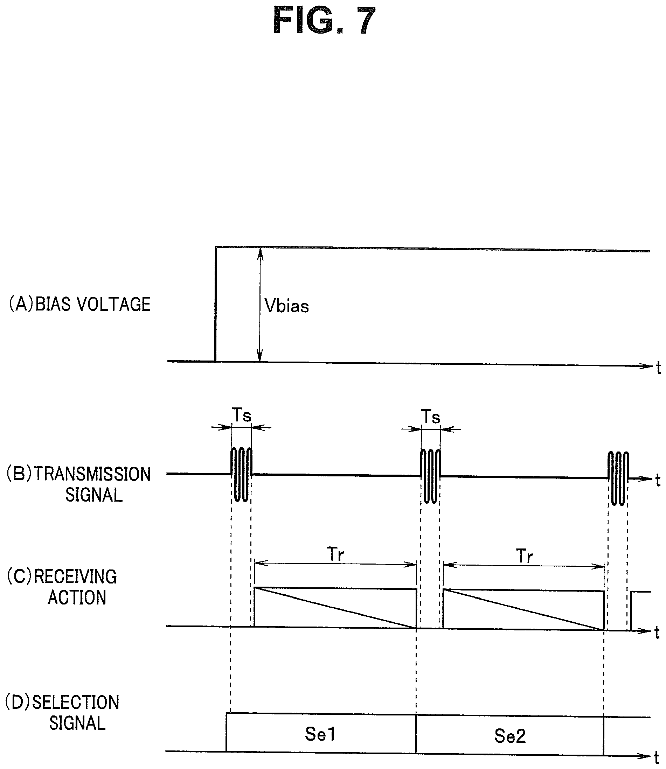

Action of the present modification is such that a cMUT used in transmission and reception changes over sequentially by a selection signal with the timing shown in Figs. 5. That is, timing according to the present modification is as shown in Figs. 7. Note that Figs. 7(A) to 7(C) are the same as Figs. 5(A) to 5(C).

As shown in Fig. 7(D), the control circuit 53B in the second observation apparatus 3B' outputs a selection signal Sel for selecting the first cMUT 11-1 during the first transmission period Ts and the first reception period Tr that come first. The first cMUT 11-1 is used for transmission and reception during the first transmission period Ts and the first reception period Tr. Note that timing for outputting the selection signal Sel or Se2 or the like may be slightly ahead of each transmission period Ts (for a transmission signal), as shown in Figs. 7.

When the first transmission period Ts and the first reception period Tr end, the control circuit 53B outputs the selection signal Se2 for selecting the second cMUT 11-2 during the second transmission period Ts and the second reception period Tr that come next. The second cMUT 11-2 is used for transmission and reception during the second transmission period Ts and the second reception period Tr.

Such actions are repeated, and the reception circuit 42B acquires a piece of image data for one frame in electronic scanning (electronic radial scanning). An ultrasound image which is produced from the piece of image data for one frame is displayed on the monitor 46. The present modification has the same effects as those of the first embodiment.

Note that although a configuration in which the bias voltage generating apparatus 21 is provided inside the first ultrasound probe 2A has been illustrated in the first embodiment, a bias voltage generating apparatus 21A may be provided inside a housing 60, from which a first ultrasound probe 2A is detachable, as shown in Fig. 8.

A second modification shown in Fig. 8 is obtained by, for example, separating the connector 9A shown in Fig. 4 on a proximal end side into two parts, a connector 9C on the ultrasound probe 2A" side and the housing 60 that incorporates the bias voltage generating apparatus 21 A having a connector receiver 60A, from which the connector 9C is detachable.

The housing 60 has a substantially identical configuration to a configuration of the connector 9A shown in Fig. 4 except for a configuration on the proximal end side.

That is, the signal line 15 connected to the motor 14 is detachable at a connector terminal P8 on the connector 9C side and a connector receiver terminal R8 of the connector receiver 60A at the housing 60 which becomes continuous with the connector terminal P8 when the connector terminal P8 comes into contact.

The signal line 18a connected to the cMUT 11A is detachable at a connector terminal P9 on the connector 9C side on the ultrasound probe 2A" side and a connector receiver terminal R9 of the connector receiver 60A at the housing 60 which becomes continuous with the connector receiver terminal R9 when the connector terminal P9 comes into contact.

Note that a structure in which the connector terminals P1 to P7 in the housing 60 are connected to the connector receiver 16A or 16B of the first or second observation apparatus 3A or 3B or the bias voltage charger 4 is identical to the structure described in the first embodiment.

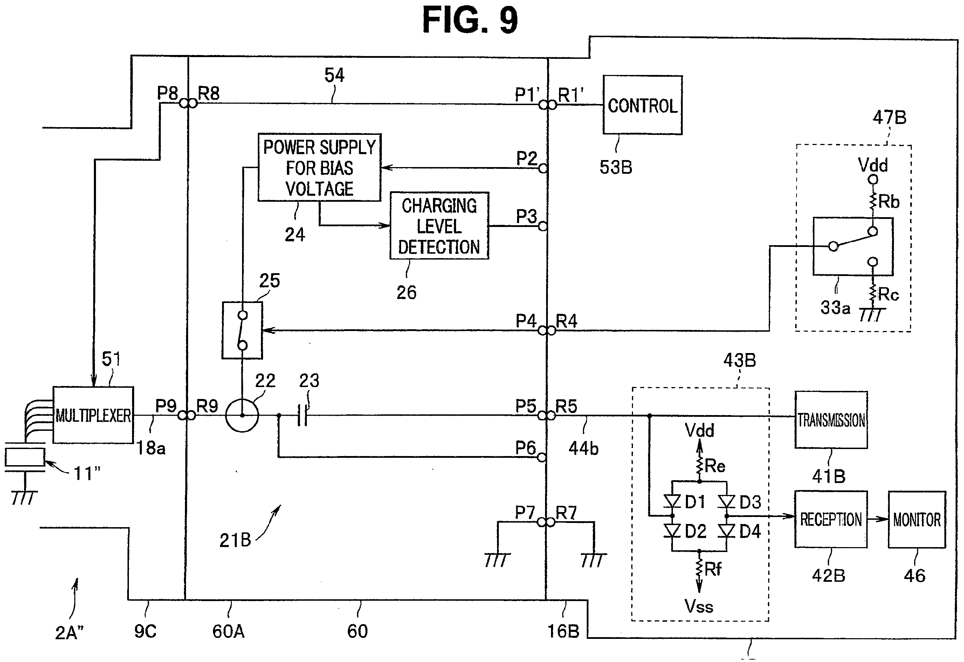

Note that although Fig. 8 shows a configuration of the bias voltage generating apparatus 21A when use of the housing 60 is applied to the first embodiment, the second modification can also be applied to the configuration in Fig. 6. Fig. 9 shows a configuration of a bias voltage generating apparatus 21B according to a third modification when the use of the housing 60 is applied to the electronic scanning-based case shown in Fig. 6.

Fig. 9 has an identical configuration to a configuration obtained by replacing the signal line 15 in Fig. 8 with the signal line 54. Note that a distal end of the signal line 54 is connected to the multiplexer 51 through the connector terminal P8 that is in contact with and continuous with the connector receiver terminal R8. A rear end of the signal line 54 is connected to the control circuit 53B in the second observation apparatus 3B through the connector receiver terminal R1' that is in contact with and continuous with the connector terminal P1'.

The configuration in Fig. 8 or Fig. 9 has the effect that use of the housing 60 incorporating the bias voltage generating apparatus 21A between the ultrasound probe 2A" and the second observation apparatus 3B for the ultrasound probe 2A" that does not have a bias voltage generating apparatus and is equipped with the cMUT 11A or a cMUT 11" allows ultrasound diagnosis or ultrasound observation through transmission and reception of ultrasound.

### (Second Embodiment)

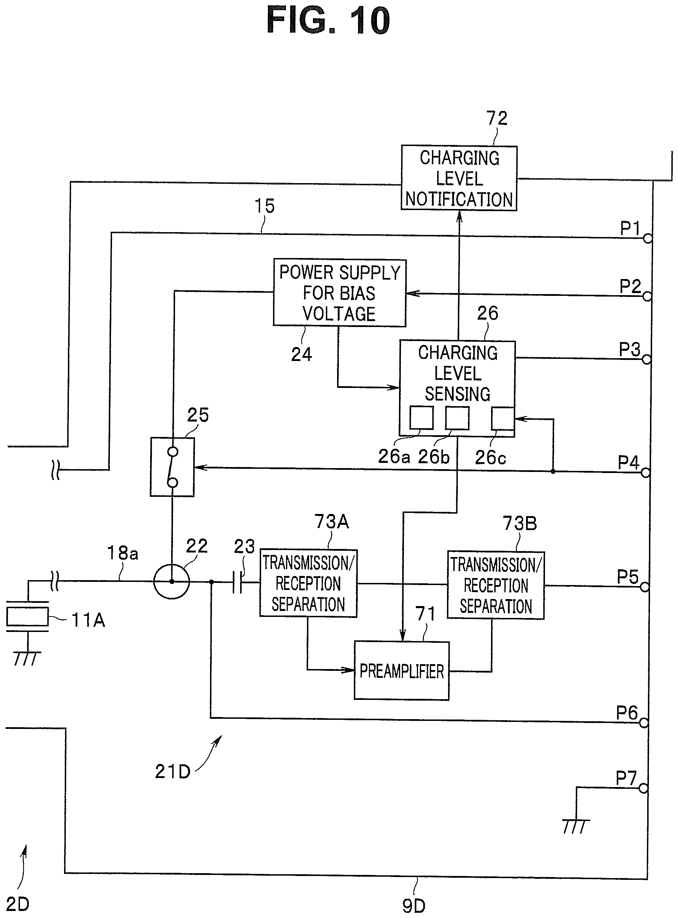

A second embodiment, embodying the present invention will be described. An ultrasound diagnostic system according to the present embodiment has a configuration obtained by further including a first ultrasound probe 2D shown in Fig. 10 in the ultrasound diagnostic system 1 in Fig. 1. In the present embodiment, the first ultrasound probe 2D is configured to include a variable gain amplifier which amplifies a received signal based on a charging level detection signal (detection information) from a charging level detecting circuit 26 and a charging level notifying portion which makes a notification of charging level.

The first ultrasound probe 2D has a connector 9D which is provided with a bias voltage generating apparatus 21D different from the bias voltage generating apparatus 21 (shown in, for example, Fig. 4) provided inside the connector 9A in the first ultrasound probe 2A in Fig. 1.

The bias voltage generating apparatus 21 D has a configuration obtained by further providing a preamplifier 71 as a variable gain amplifier, a gain of which is changed by a charging level detection signal from the charging level detecting circuit 26, and a charging level notifying circuit 72, and two transmission/reception separating circuits 73A and 73B which are provided on a signal line 18a in the bias voltage generating apparatus 21 shown in Fig. 4. Note that the preamplifier 71 is placed between the two transmission/reception separating circuits 73A and 73B to amplify a received signal with a gain of 1 or more when the received signal is inputted through the transmission/reception separating circuit 73A. The amplified signal is outputted to a connector terminal P6 or P5 side serving as a transmission/reception terminal through the transmission/reception separating circuit 73B. The two transmission/reception separating circuits 73A and 73B may have an identical configuration to, for example, the configuration of the transmission/reception separating circuit 43B in Fig. 4.

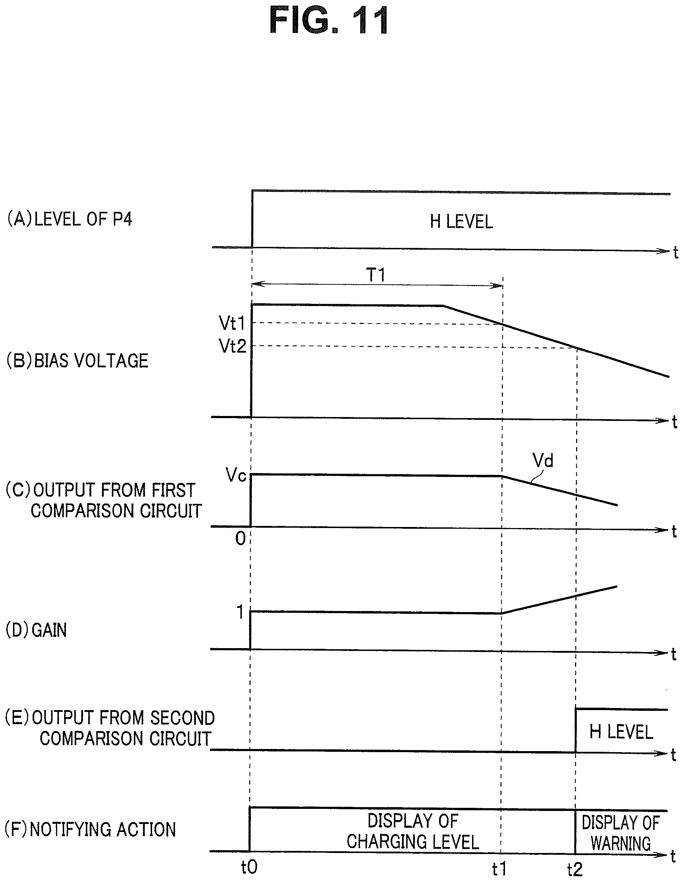

In addition to the charging level detecting function described in the first embodiment, the charging level detecting circuit 26 according to the present embodiment detects, using a first comparison circuit 26a, whether a bias voltage outputted from a power supply circuit 24 for bias voltage has become less than a lower limit value Vt1 of an appropriate bias voltage range set in advance and has a second comparison circuit 26b which detects whether the bias voltage has become not more than a second voltage value Vt2 lower than the lower limit value Vt1. Note that the first comparison circuit 26a outputs a signal corresponding to H level (e.g., a signal at a level Vc) if a result of a comparison with the lower limit value Vt1 shows that the bias voltage is not less than the lower limit value Vt1 and outputs a first detection signal at a level Vd proportional to the bias voltage less than the lower limit value Vt1 if the bias voltage is less than the lower limit value Vt1, as shown in Fig. 11(C).

The charging level detecting circuit 26 also has a third comparison circuit 26c which judges whether level of a signal applied to a connector terminal P4 is either H or L, i.e., whether the level is H level that turns on a bias switch 25 (or the connector 9C is connected to a second observation apparatus 3B).

The charging level detecting circuit 26 controls action of the preamplifier 71 only if the level of the signal applied to the connector terminal P4 is H level (i.e., if the connector 9C is connected to the second observation apparatus 3B). If the level of the signal applied to the connector terminal P4 is L level, the charging level detecting circuit 26 does not control the action of the preamplifier 71.

If a bias voltage outputted from the power supply circuit 24 for bias voltage is less than the lower limit value Vt1 of the appropriate bias voltage range set in advance, sensitivity to a received signal which is received and produced by a cMUT 11A is lower than when the bias voltage is within the appropriate bias voltage range.

For the reason, in the present embodiment, the charging level detecting circuit 26 changes a voltage to be applied to a gain control terminal so as to make a gain of the preamplifier 71 higher than a gain of 1 (which is the gain when the bias voltage is within the appropriate bias voltage range), if a bias voltage outputted from the power supply circuit 24 for bias voltage becomes less than the lower limit value Vt1. In the present embodiment, a drop in sensitivity to a received signal caused when the bias voltage falls below the appropriate range is compensated for through an increase in the gain of the preamplifier 71.

The charging level detecting circuit 26 outputs a detection signal relating to charging level of the power supply circuit 24 for bias voltage to the charging level notifying circuit 72. The charging level notifying circuit 72 notifies a user of the charging level by displaying the charging level on a display.

The charging level detecting circuit 26 outputs a second detection signal to the charging level notifying circuit 72 when a bias voltage outputted from the power supply circuit 24 for bias voltage becomes not more than the second voltage value Vt2. When the second detection signal is inputted, the charging level notifying circuit 72 displays the charging level and displays a message prompting charging to notify the user of the message prompting charging.

Note that, if the connector 9D is connected to a first observation apparatus 3A in the present embodiment, a bias voltage generating circuit 49 of the first observation apparatus 3A constantly outputs a bias voltage within the appropriate range, and sensitivity correction by the preamplifier 71 is not performed.

Other components are the same as the components in the first embodiment. Action of the present embodiment will be described. If the first ultrasound probe 2A is used in the present embodiment, a same action as the action of the first embodiment is performed. A description of the case will be omitted, and a main action when the first ultrasound probe 2D is used will be described. A case where transmission and reception of ultrasound are performed with the first ultrasound probe 2D connected to the second observation apparatus 3B will also be described.

Figs. 11 show timing charts for explaining action in the case. Each horizontal axis in Figs. 11 indicates t. Assume that actions of the bias voltage generating apparatus 21 D and the like start at time t0.

As shown in Fig. 11(A), the connector terminal P4 is at H level from time t0 onward. When the bias switch 25 is turned on, the charging level detecting circuit 26 acts to control action of the preamplifier 71 and the charging level notifying circuit 72.

During a period T1 when the power supply circuit 24 for bias voltage outputs a bias voltage within the appropriate range, as shown in Fig. 11 (B), the first comparison circuit 26a of the charging level detecting circuit 26 applies a first detection signal at the fixed level Vc to the gain control terminal of the preamplifier 71, as shown in Fig. 11(C). In the state, the gain of the preamplifier 71 is set to 1, as shown in Fig. 11 (D).

In the state, the bias voltage generating apparatus 21D performs a substantially identical action to the action of the first embodiment where the first ultrasound probe 2A is connected to the second observation apparatus 3B.

The charging level notifying circuit 72 displays the charging level on the display to make a notification of the charging level, as shown in Fig. 11 (F).

When the charging level of the power supply circuit 24 for bias voltage drops, and a bias voltage outputted from the power supply circuit 24 for bias voltage becomes less than the bias voltage lower limit value Vt1 of the appropriate range (at time t1), the first comparison circuit 26a applies (outputs) a first detection signal at the level Vd that changes in proportion to a drop from the lower limit value Vt1 to the gain control terminal of the preamplifier 71, as shown in Fig. 11(C).

The preamplifier 71 enters a state acting to amplify a received signal with a gain (higher than 1) which rises in proportion to the amount of change from the fixed level Vc.

When the bias voltage outputted from the power supply circuit 24 for bias voltage drops further and becomes not more than the second voltage value Vt2 (at time t2), the second comparison circuit 26b outputs a second detection signal (at, for example, H level) to the charging level notifying circuit 72, as shown in Fig. 11 (E).

When the second detection signal is inputted to the charging level notifying circuit 72, the charging level notifying circuit 72 displays, on the display, a warning that the charging level of the power supply circuit 24 for bias voltage has dropped to a level requiring charging and makes a notification so as to prompt a user to perform charging. That is, the charging level notifying circuit 72 makes a notification through display of a warning, as shown in Fig. 11 (F). With the display of the warning, the user recognizes a state requiring charging.

Note that, if transmission and reception of ultrasound is performed with the first ultrasound probe 2D connected to the first observation apparatus 3A, the preamplifier 71 does not act, and the charging level notifying circuit 72 acts in a same manner as in the above description.

According to the present embodiment with the above-described action, a drop in sensitivity to a received signal can be compensated for even if a bias voltage outputted from the power supply circuit 24 for bias voltage becomes less than the bias voltage lower limit value Vt1 of the appropriate range, in addition to the same effects as those of the first embodiment.

Note that the ultrasound diagnostic system according to the present embodiment has been described in a context of a case where the ultrasound probe 2D is further provided in the ultrasound diagnostic system 1 in Fig. 1. An electronic scanning-based ultrasound probe provided with the bias voltage generating apparatus 21D shown in Fig. 10 can be provided in the ultrasound diagnostic system 1' shown in Fig. 6 as well.

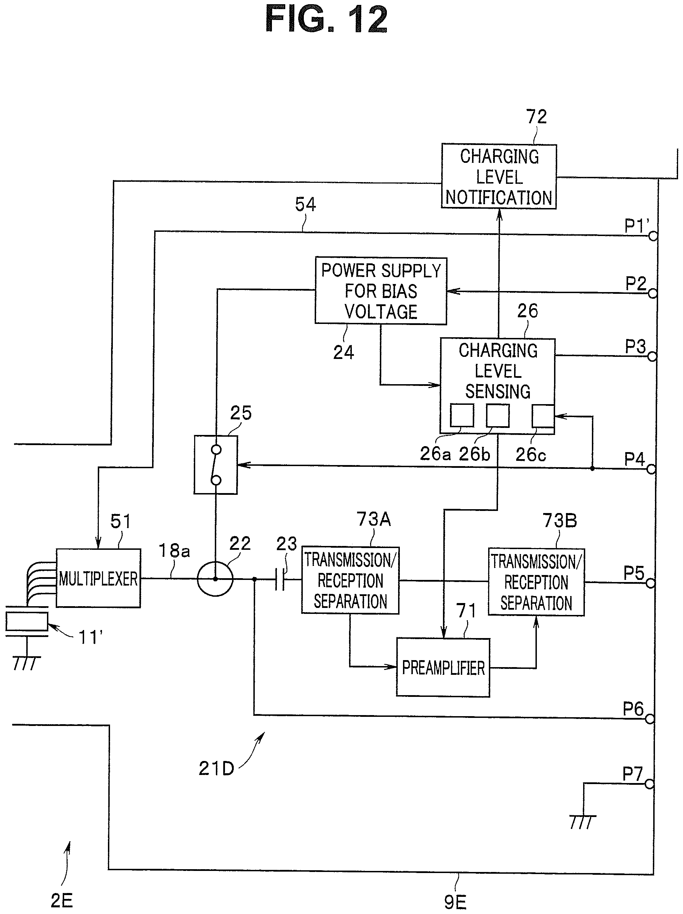

Fig. 12 shows a configuration of a main portion of an electronic scanning-based first ultrasound probe 2E in an ultrasound diagnostic system according to a modification of the second embodiment. The ultrasound diagnostic system according to the present modification is obtained by further including the electronic scanning-based first ultrasound probe 2E shown in Fig. 12 in the ultrasound diagnostic system 1' shown in Fig. 6.

The electronic scanning-based first ultrasound probe 2E is obtained by replacing the signal line 15 with a signal line 54 and the connector 9D with a connector 9E, in the ultrasound probe 2D in Fig. 10. A distal end of the signal line 54 is connected to a multiplexer 51 while a rear end is connected to a connector terminal P1'. Other components are the same as the components described with reference to Fig. 10. The present modification has substantially same operations and/or effects as those of the second embodiment except that the present modification is different in an ultrasound scanning method from the second embodiment.

Although a configuration in which the bias voltage generating apparatus 21D is provided in the connector 9D of the ultrasound probe 2D is shown in Fig. 10, the bias voltage generating apparatus 21D can be configured, through same modification as the modification of the configuration in Fig. 4 in the manner in Fig. 8, such that the connector of the ultrasound probe 2D is detachable from the bias voltage generating apparatus 21D. The modification can also be applied to the ultrasound probe 2E in Fig. 12.

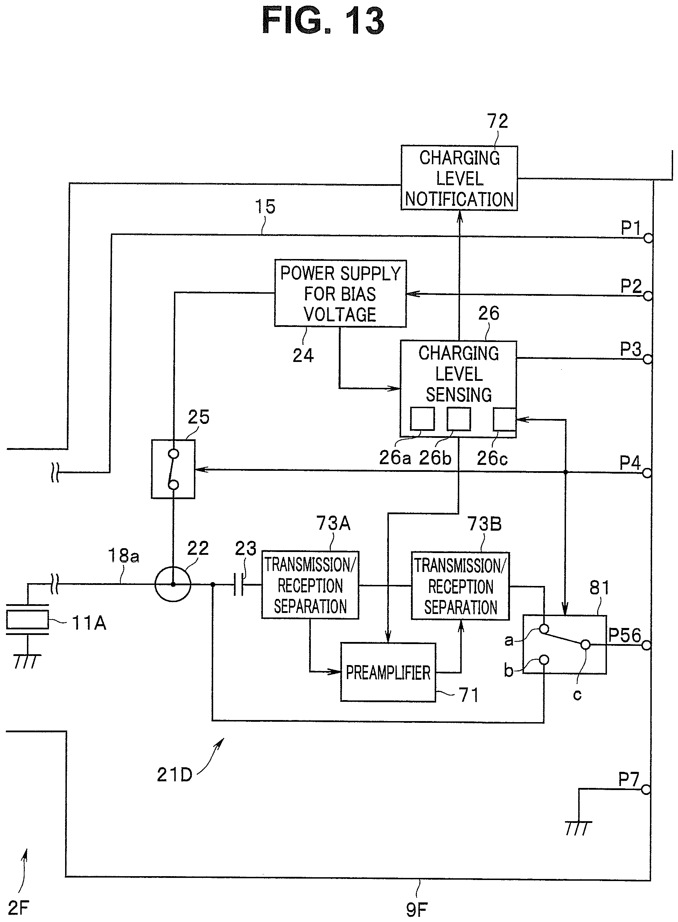

Note that a configuration has been described in the above-described embodiment and the like in which connector terminals detachable from the connector receiver terminal R6 serving as an ultrasound transmission/reception terminal of the first observation apparatus 3A and the connector receiver terminal R5 serving as an ultrasound transmission/reception terminal of the second observation apparatus 3B are not a common one but are the two connector terminals P6 and P5. In contrast, as shown in Fig. 13, the connector terminals P5 and P6 may be integrated into one common connector terminal P56 by providing a change-over switch 81. Fig. 13 shows an example of a configuration of an ultrasound probe 2F which is a modification of the ultrasound probe 2D in Fig. 10.

The ultrasound probe 2F has a connector 9F which is obtained by providing the change-over switch 81 on a signal line between the connector terminal P5 and the transmission/reception separating circuit 73B and replacing the connector terminal P5 with the connector terminal P56, in the connector 9D of the ultrasound probe 2D in Fig. 10.

The connector terminal P56 is connected to a common contact c of the change-over switch 81, the transmission/reception separating circuit 73B and a signal line extending from a junction of a bias voltage superimposing circuit 22 and a capacitor 23 to the connector terminal P6 (in Fig. 10) are connected to contacts a and b, respectively, and change-over of the change-over switch 81 is controlled according to signal level of the connector terminal P4.

More specifically, if level of a signal applied to the connector terminal P4 is at H level that turns on the bias switch 25, i.e., if the second observation apparatus 3B is connected to the connector 9F, the common contact c is made continuous with the contact a. On the other hand, if the level of the signal applied to the connector terminal P4 is at L level, i.e., if the first observation apparatus 3A is connected to the connector 9F, the common contact c is made continuous with the contact b.

Fig. 13 does not have the connector terminal P6. The connector receiver terminal R5 of the second observation apparatus 3B shown in Fig. 1, 4, or the like is detachably connected to the connector terminal P56, and the connector receiver terminal R6 of the first observation apparatus 3A shown in Fig. 1, 3, or the like is detachably connected to the connector terminal P56.

Other components are the same as the components described with reference to Fig. 10 and the like. According to the present modification, the common connector terminal P56 can be connected to the respective ultrasound transmission/reception terminals of the first observation apparatus 3A and the second observation apparatus 3B. It is thus possible to reduce the number of connector terminals. Note that although Fig. 13 shows an application to the configuration in Fig. 10, the present modification can also be applied to the first embodiment, the second embodiment, and the like.

Note that configurations according to the embodiments including the modifications are not limited to the configurations shown in the drawings. A configuration obtained by removing a component from the ultrasound diagnostic system 1 or 1' described with reference to Fig. 1, 6, or the like as needed also belongs to the present invention. For example, the ultrasound diagnostic system 1 shown in Fig. 1 can also be configured not to have the second ultrasound probe 2B.

Alternatively, the ultrasound diagnostic system 1 shown in Fig. 1 can also be configured not to have the first observation apparatus 3A. Although the configuration in Fig. 1 has been described, a same applies to the configuration in Fig. 6.

Although cases where the multiplexer 51 or 51B is provided inside an ultrasound probe such as the ultrasound probe 2A' or 2B' have been described in the configuration examples, the multiplexer 51 or 51B may be provided inside an observation apparatus such as the observation apparatus 3A' or 3B', instead of being provided inside an ultrasound probe. In the case, a bias voltage from the power supply circuit 24 for bias voltage may be superimposed on signal lines connected to the individual cMUTs 11-j, respectively, via the bias switch 25 (that is controlled so as to be turned on or off by a switch control signal).

## Claims

1. A bias voltage generating apparatus (21D) adapted to be placed outside an ultrasound observation apparatus (3A, 3B) and to be used together with the ultrasound observation apparatus (3A, 3B), the ultrasound observation apparatus (3A, 3B) incorporating a transmission circuit (41) generating a transmission signal and a reception circuit (42) performing processing on a received signal, for transmitting/receiving ultrasound to/from a subject using a capacitive transducer (11A), comprising:
a power supply circuit for bias voltage (24) which includes a chargeable secondary battery (24a) for producing a bias voltage to be applied to the capacitive transducer (11A) and produces the bias voltage; and
a bias voltage superimposing portion (22) which superimposes the bias voltage on the transmission signal outputted to outside the ultrasound observation apparatus (3A, 3B) and on the received signal to be inputted inside the ultrasound observation apparatus (3A, 3B),
**characterized by**
a charging level detecting portion (26) which detects a charging level of the secondary battery (24a) constituting the power supply circuit for bias voltage (24);
a charging level notifying portion (72) which notifies charging level information detected by the charging level detecting portion (26); and
a variable gain amplifier (71) which varies a gain for amplifying the received signal to be inputted inside the ultrasound diagnostic observation apparatus based on the charging level detected by the charging level detecting portion (26), wherein
when the bias voltage has become less than a lower limit value of an appropriate bias voltage range, the charging level detecting portion (26) compensates for a drop in sensitivity to the received signal caused when the bias voltage falls below the appropriate range through an increase in the gain of the variable gain amplifier (71).

2. The bias voltage generating apparatus (21D) according to claim 1, wherein
the bias voltage generating apparatus (21D) is placed in an ultrasound probe (2D, 2E) which is equipped with the capacitive transducer (11A).

3. The bias voltage generating apparatus (21D) according to claim 1, wherein
the bias voltage generating apparatus (21D) is detachably connected to an ultrasound probe (2D, 2E) which is equipped with the capacitive transducer (11A) and the ultrasound observation apparatus (3A, 3B).

4. The bias voltage generating apparatus (21D) according to claim 1, wherein
the bias voltage generating apparatus (21D) further comprises a switch (25) which turns on or off superimposition of the bias voltage generated by the power supply circuit for bias voltage (24) on the bias voltage superimposing portion (22) and a control terminal which controls the switch (25) from an outside to be turned on or off.

5. The bias voltage generating apparatus (21D) according to claim 1, wherein
the power supply circuit for bias voltage (24) has the secondary battery (24a) and a DC/DC converter (24b) which raises a DC voltage of the secondary battery (24a) and produces the bias voltage.

6. The bias voltage generating apparatus (21D) according to claim 2, wherein
the bias voltage generating apparatus (21D) is placed in a connector in the probe (2D, 2E), the connector being detachably connected to the ultrasound observation apparatus (3A, 3B).

7. The bias voltage generating apparatus (21D) according to claim 2, wherein
the bias voltage generating apparatus (21D) has a plurality of first connector terminals which are detachably connected to a plurality of connector receiver terminals of a connector receiver of the ultrasound observation apparatus (3A, 3B) and has a plurality of second connector terminals which are detachably connected to connector receiver terminals of a connector receiver of a charger (4) generating a charging voltage for charging the secondary battery (24a).

8. The bias voltage generating apparatus (21D) according to claim 4, wherein
the bias voltage generating apparatus (21D) further has a charger (4) which is detachably connected to the bias voltage generating apparatus (21D) and generates a charging voltage for charging the secondary battery (24a), and the charger (4) has a switch control circuit (33) which sets the switch to off via the control terminal if the charger(4) is connected to the bias voltage generating apparatus (21D).

9. An ultrasound diagnostic system (1, 1B) comprising:
an ultrasound probe (2D, 2E) which is equipped with a capacitive transducer (11A);
an ultrasound observation apparatus (3A, 3B) which incorporates a transmission circuit (41) generating a transmission signal for causing the capacitive transducer (11A) to generate ultrasound, a reception circuit (42) performing processing on a received signal outputted from the capacitive transducer (11A) upon reception of ultrasound, and a transmission/reception separating circuit (43) which separates the transmission signal from the received signal; and
a bias voltage generating apparatus (21D) which is incorporated in or detachably connected to the ultrasound probe (2D, 2E) and includes a power supply circuit for bias voltage (24) including a chargeable secondary battery (24a) for producing a bias voltage to be applied to the capacitive transducer (11A) and producing the bias voltage and a bias voltage superimposing portion (22) superimposing the bias voltage on the transmission signal outputted to outside the ultrasound observation apparatus (3A, 3B) and on the received signal to be inputted inside the ultrasound observation apparatus (3A, 3B),
**characterized by**
a charging level detecting portion (26) which detects a charging level of the secondary battery (24a) constituting the power supply circuit for bias voltage (24);
a charging level notifying portion (72) which notifies charging level information detected by the charging level detecting portion (26); and
a variable gain amplifier (71) which varies a gain for amplifying the received signal to be inputted inside the ultrasound diagnostic observation apparatus based on the charging level detected by the charging level detecting portion (26), wherein
when the bias voltage has become less than a lower limit value of an appropriate bias voltage range, the charging level detecting portion (26) compensates for a drop in sensitivity to the received signal caused when the bias voltage falls below the appropriate range through an increase in the gain of the variable gain amplifier (71).

10. The ultrasound diagnostic system (1, 1B) according to claim 9, wherein
the bias voltage generating apparatus (21D) further includes a switch (25) which turns on or off superimposition of the bias voltage generated by the power supply circuit for bias voltage (24) on the bias voltage superimposing portion (22) and a control terminal which controls the switch (25) from outside the bias voltage generating apparatus (21D) to be turned on or off.

11. The ultrasound diagnostic system (1, 1B) according to claim 10, wherein
the system (1, 1B) further has a second observation apparatus (3B) which incorporates a switch control circuit (47B) generating, for the switch (25), a switch control signal for turning off superimposition of the bias voltage, a second transmission circuit (41B) generating a second transmission signal for causing the capacitive transducer (11A) to generate ultrasound, a second reception circuit (42B) performing signal processing on a received signal outputted from the capacitive transducer (11A), and a bias voltage circuit generating a second bias voltage to be superimposed on a transmission signal from the second transmission circuit (41B) and the received signal inputted to the second reception circuit (42B) and superimposing the second bias voltage, in addition to the ultrasound observation apparatus, to which a received signal with the superimposed bias voltage generated by the bias voltage generating apparatus (21D) is inputted.

12. The ultrasound diagnostic system (1, 1B) according to claim 9, wherein
the power supply circuit for bias voltage (24) has the secondary battery (24a) and a DC/DC converter (24b) which raises a DC voltage of the secondary battery (24a) and produces the bias voltage.

13. The ultrasound diagnostic system (1, 1B) according to claim 10, wherein
the system (1, 1B) further has a charger (4) which is detachably connected to the bias voltage generating apparatus (21D) and generates a charging voltage for charging the secondary battery (24a), and the charger (4) has a switch control circuit (33) which sets the switch to off via the control terminal if the charger (4) is connected to the bias voltage generating apparatus (21D).

14. The ultrasound diagnostic system (1, 1B) according to claim 10, wherein
the ultrasound probe (2D, 2E) is composed of a mechanical scanning-based ultrasound probe which mechanically performs ultrasound scanning using the capacitive transducer (11A).

15. The ultrasound diagnostic system (1, 1B) according to claim 10, wherein
the ultrasound probe (2D, 2E) has an array of a plurality of capacitive transducers as the capacitive transducer and is composed of an electronic scanning-based ultrasound probe which electronically performs ultrasound scanning by driving the array of the capacitive transducers.

## Patentansprüche

1. Vorspannungserzeugungsgerät (21D), das dazu eingerichtet ist, außerhalb eines Ultraschallbeobachtungsgeräts (3A, 3B) platziert und gemeinsam mit dem Ultraschallbeobachtungsgerät (3A, 3B) verwendet zu werden, wobei das Ultraschallbeobachtungsgerät (3A, 3B) eine Sendeschaltung (41), die ein Sendesignal erzeugt, und eine Empfangsschaltung (42) umfasst, die eine Verarbeitung an einem empfangenen Signal durchführt, um unter Verwendung eines kapazitiven Wandlers (11A) Ultraschall zu/von einem Subjekt zu senden/empfangen, umfassend:
eine Energiezufuhrschaltung für eine Vorspannung (20), die eine wiederaufladbare sekundäre Batterie (24a) zum Erzeugen einer Vorspannung umfasst, die dazu vorgesehen ist, an den kapazitiven Wandler (11A) angelegt zu werden, und die die Vorspannung erzeugt; und
einen Vorspannungsüberlagerungsabschnitt (22) der die Vorspannung dem nach außerhalb des Ultraschallbeobachtungsgeräts (3A, 3B) ausgegebenen Sendesignal und dem innerhalb des Ultraschallbeobachtungsgeräts (3A, 3B) einzugebenden empfangenen Signal überlagert,
**gekennzeichnet durch**
einen Ladeniveauerfassungsabschnitt (26), der ein Ladeniveau der sekundären Batterie (24a) erfasst, die die Energiezufuhrschaltung für eine Vorspannung (24) bildet;
einen Ladeniveauanzeigeabschnitt (72), der eine von dem Ladeniveauerfassungsabschnitt (26) erfasste Ladeniveauinformation anzeigt; und
einen variablen Verstärker (71), der eine Verstärkung zum Verstärken des innerhalb des Ultraschallbeobachtungsgeräts (3A, 3B) einzugebenden empfangenen Signals auf der Basis des von dem Ladeniveauerfassungsabschnitt (26) erfassten Ladeniveaus variiert, wobei
wenn die Vorspannung kleiner geworden ist als ein unterer Grenzwert eines geeigneten Vorspannungbereichs, der Ladeniveauerfassungsabschnitt (26) einen Empfindlichkeitsabfall für das empfangene Signal, der verursacht wird, wenn die Vorspannung unter den geeigneten Bereich fällt, **durch** eine Zunahme der Verstärkung des variablen Verstärker (71) kompensiert.

2. Vorspannungserzeugungsgerät (21D) nach Anspruch 1, bei dem
das Vorspannungserzeugungsgerät (21D) in einer Ultraschallsonde (2D, 2E) platziert ist, die mit dem kapazitiven Wandler (11A) ausgestattet ist.

3. Vorspannungserzeugungsgerät (21D) nach Anspruch 1, bei dem
das Vorspannungserzeugungsgerät (21D) lösbar mit einer Ultraschallsonde (2D, 2E), die mit dem kapazitiven Wandler (11A) ausgestattet ist, und dem Ultraschallbeobachtungsgerät (3A, 3B) verbunden ist.

4. Vorspannungserzeugungsgerät (21D) nach Anspruch 1, bei dem
das Vorspannungserzeugungsgerät (21D) ferner einen Schalter (25), der eine Überlagerung der von der Energiezufuhrschaltung für eine Vorspannung (24) erzeugten Vorspannung über den Vorspannungsüberlagerungsabschnitt (22) an- oder ausschaltet, und einen Steueranschluss umfasst, der den Schalter (25) von außerhalb steuert, um an- oder ausgeschaltet zu werden.

5. Vorspannungserzeugungsgerät (21D) nach Anspruch 1, bei dem
die Energiezufuhrschaltung für eine Vorspannung (24) die sekundäre Batterie (24a) und einen Gleichspannungswandler (24b) aufweist, der eine Gleichspannung der sekundären Batterie (24a) erhöht und die Vorspannung erzeugt.

6. Vorspannungserzeugungsgerät (21D) nach Anspruch 2, bei dem
das Vorspannungserzeugungsgerät (21D) in einem Verbinder in der Sonde (2D, 2E) platziert ist, wobei der Verbinder lösbar mit dem Ultraschallbeobachtungsgerät (3A, 3B) verbunden ist.

7. Vorspannungserzeugungsgerät (21D) nach Anspruch 2, bei dem
das Vorspannungserzeugungsgerät (21D) eine Mehrzahl von ersten Verbinderanschlüssen aufweist, die lösbar mit einer Mehrzahl von Verbinderempfängeranschlüssen eines Verbinderempfängers des Ultraschallbeobachtungsgeräts (3A, 3B) verbunden sind, und eine Mehrzahl von zweiten Verbinderanschlüssen aufweist, die lösbar mit Verbinderempfängeranschlüssen eines Verbinderempfängers eines Ladegeräts (4) verbunden sind, welches eine Ladespannung zum Laden der sekundären Batterie (24a) erzeugt.

8. Vorspannungserzeugungsgerät (21D) nach Anspruch 4, bei dem
das Vorspannungserzeugungsgerät (21D) ferner ein Ladegerät (4) aufweist, das lösbar mit dem Vorspannungserzeugungsgerät (21D) verbunden ist und eine Ladespannung zum Laden der sekundären Batterie (24a) erzeugt, und das Ladegerät (4) eine Schaltersteuerschaltung (33) aufweist, die über den Steueranschluss den Schalter ausschaltet, wenn das Ladegerät (4) mit dem Vorspannungserzeugungsgerät (21D) verbunden ist.

9. Ultraschalldiagnosesystem (1, 1B), das umfasst:
eine Ultraschallsonde (2D, 2E), die mit einem kapazitiven Wandler (11A) ausgestattet ist;
ein Ultraschallbeobachtungsgerät (3A, 3B), das eine Sendeschaltung (41), die ein Sendesignal erzeugt, um den kapazitiven Wandler (11A) zur Erzeugung von Ultraschall zu veranlassen, eine Empfangsschaltung (42), die eine Verarbeitung an einem empfangenen Signal durchführt, das von dem kapazitiven Wandler (11A) beim Empfang von Ultraschall ausgegeben wird, und eine Sende/Empfangs-Trennschaltung umfasst, die das Sendesignal von dem empfangenen Signal trennt; und
ein Vorspannungserzeugungsgerät (21D), das in die Ultraschallsonde (2D, 2E) integriert oder lösbar damit verbunden ist und eine Energiezufuhrschaltung für eine Vorspannung (20), die eine wiederaufladbare sekundäre Batterie (24a) zum Erzeugen einer Vorspannung umfasst, die dazu vorgesehen ist, an den kapazitiven Wandler (11A) angelegt zu werden, und die die Vorspannung erzeugt, und einen Vorspannungsüberlagerungsabschnitt (22) umfasst, der die Vorspannung dem nach außerhalb des Ultraschallbeobachtungsgeräts (3A, 3B) ausgegebenen Sendesignal und dem innerhalb des Ultraschallbeobachtungsgeräts (3A, 3B) einzugebenden empfangenen Signal überlagert,
**gekennzeichnet durch**
einen Ladeniveauerfassungsabschnitt (26), der ein Ladeniveau der sekundären Batterie (24a) erfasst, die die Energiezufuhrschaltung für eine Vorspannung (24) bildet;
einen Ladeniveauanzeigeabschnitt (72), der eine von dem Ladeniveauerfassungsabschnitt (26) erfasste Ladeniveauinformation anzeigt; und
einen variablen Verstärker (71), der eine Verstärkung zum Verstärken des innerhalb des Ultraschallbeobachtungsgeräts einzugebenden empfangenen Signals auf der Basis des von dem Ladeniveauerfassungsabschnitt (26) erfassten Ladeniveaus variiert, wobei
wenn die Vorspannung kleiner geworden ist als ein unterer Grenzwert eines geeigneten Vorspannungbereichs, der Ladeniveauerfassungsabschnitt (26) einen Empfindlichkeitsabfall für das empfangene Signal, der verursacht wird, wenn die Vorspannung unter den geeigneten Bereich fällt, **durch** eine Zunahme der Verstärkung des variablen Verstärker (71) kompensiert.

10. Ultraschalldiagnosesystem (1, 1B) nach Anspruch 9, bei dem
das Vorspannungserzeugungsgerät (21D) ferner einen Schalter (25), der eine Überlagerung der von der Energiezufuhrschaltung für eine Vorspannung (24) erzeugten Vorspannung über den Vorspannungsüberlagerungsabschnitt (22) an- oder ausschaltet, und einen Steueranschluss umfasst, der den Schalter (25) von außerhalb steuert, um an- oder ausgeschaltet zu werden.

11. Ultraschalldiagnosesystem (1, 1B) nach Anspruch 10, bei dem
das System (1, 1B) zusätzlich zu dem Ultraschallbeobachtungsgerät, in das ein empfangenes Signal mit der durch das Vorspannungserzeugungsgerät (21D) erzeugten überlagerten Vorspannung eingegeben wird, ferner ein zweites Beobachtungsgerät (3B) aufweist, das eine Schaltersteuerschaltung (47B), welche für den Schalter (25) ein Schaltersteuersignal zum Abschalten einer Überlagerung der Vorspannung erzeugt, eine zweite Sendeschaltung (41B), die ein zweites Sendesignal erzeugt, um den kapazitiven Wandler (11A) zur Erzeugung von Ultraschall zu veranlassen, eine zweite Empfangsschaltung (42B), die an einem von dem kapazitiven Wandler (11A) ausgegebenen empfangenen Signal eine Signalverarbeitung durchführt, und eine Vorspannungsschaltung umfasst, die eine zweite Vorspannung erzeugt, die dazu vorgesehen ist, einem Sendesignal von der zweiten Sendeschaltung (41B) und dem in die zweite Empfangsschaltung (42B) eingegebenen und der zweiten Vorspannung überlagerten empfangenen Signal überlagert zu werden.

12. Ultraschalldiagnosesystem (1, 1B) nach Anspruch 9, bei dem
die Energiezufuhrschaltung für eine Vorspannung (24) die sekundäre Batterie (24a) und einen Gleichspannungswandler (24b) aufweist, der eine Gleichspannung der sekundären Batterie (24a) erhöht und die Vorspannung erzeugt.

13. Ultraschalldiagnosesystem (1, 1B) nach Anspruch 10, bei dem
das Vorspannungserzeugungsgerät (21D) ferner ein Ladegerät (4) aufweist, das lösbar mit dem Vorspannungserzeugungsgerät (21D) verbunden ist und eine Ladespannung zum Laden der sekundären Batterie (24a) erzeugt, und das Ladegerät (4) eine Schaltersteuerschaltung (33) aufweist, die über den Steueranschluss den Schalter ausschaltet, wenn das Ladegerät (4) mit dem Vorspannungserzeugungsgerät (21D) verbunden ist.

14. Ultraschalldiagnosesystem (1, 1B) nach Anspruch 10, bei dem
die Ultraschallsonde (2D, 2E) aus einer mechanischen abtastbasierten Ultraschallsonde besteht, die unter Verwendung des kapazitiven Wandlers (11A) eine Ultraschallabtastung mechanisch durchführt.

15. Ultraschalldiagnosesystem (1, 1B) nach Anspruch 10, bei dem
die Ultraschallsonde (2D, 2E) eine Anordnung aus einer Mehrzahl von kapazitiven Wandlern als den kapazitiven Wandler aufweist und aus einer elektronischen abtastbasierten Ultraschallsonde besteht, die durch Antreiben der Anordnung aus kapazitiven Wandlern eine Ultraschallabtastung elektronisch durchführt.

## Revendications

1. Appareil (21D) de génération de tension de polarisation adapté à être placé à l'extérieur d'un appareil (3A, 3B) d'observation par ultrasons et à être utilisé ensemble avec l'appareil (3A, 3B) d'observation par ultrasons, l'appareil (3A, 3B) d'observation par ultrasons incorporant un circuit (41) de transmission générant un signal de transmission et un circuit (42) de réception exécutant un traitement sur un signal reçu, pour transmettre/recevoir des ultrasons à un/d'un sujet en utilisant un transducteur (11A) capacitif, comprenant :
un circuit d'alimentation électrique pour une tension (24) de polarisation qui inclut une batterie secondaire (24a) rechargeable pour produire une tension de polarisation destinée à être appliquée au transducteur (11A) capacitif et produit la tension de polarisation ; et
une partie (22) de superposition de tension de polarisation qui superpose la tension de polarisation sur le signal de transmission délivré en sortie jusqu'à l'extérieur de l'appareil (3A, 3B) d'observation par ultrasons et sur le signal reçu destiné à être entré à l'intérieur de l'appareil (3A, 3B) d'observation par ultrasons,
**caractérisé par**
une partie (26) de détection de niveau de charge qui détecte un niveau de charge de la batterie secondaire (24a) constituant le circuit d'alimentation électrique pour une tension (24) de polarisation ;
une partie (72) de notification de niveau de charge qui notifie une information de niveau de charge détecté par la partie (26) de détection de niveau de charge ; et
un amplificateur (71) à gain variable qui fait varier un gain pour amplifier le signal reçu destiné à être entré à l'intérieur de l'appareil d'observation de diagnostic par ultrasons sur la base du niveau de charge détecté par la partie (26) de détection de niveau de charge, dans lequel
lorsque la tension de polarisation est devenue inférieure à une valeur limite inférieure d'une plage appropriée de tension de polarisation, la partie (26) de détection de niveau de charge compense une chute de sensibilité au signal reçu causée lorsque la tension de polarisation chute en-dessous de la plage appropriée par l'intermédiaire d'une augmentation du gain de l'amplificateur (71) à gain variable.

2. Appareil (21D) de génération de tension de polarisation selon la revendication 1, dans lequel
l'appareil (21D) de génération de tension de polarisation est placé dans une sonde (2D, 2E) à ultrasons qui est équipée avec le transducteur (11A) capacitif.

3. Appareil (21D) de génération de tension de polarisation selon la revendication 1, dans lequel
l'appareil (21D) de génération de tension de polarisation est connecté de manière détachable à une sonde (2D, 2E) à ultrasons qui est équipée avec le transducteur (11A) capacitif et l'appareil (3A, 3B) d'observation par ultrasons

4. Appareil (21D) de génération de tension de polarisation selon la revendication 1, dans lequel
l'appareil (21D) de génération de tension de polarisation comprend en outre un commutateur (25) qui active ou désactive la superposition de la tension de polarisation générée par le circuit d'alimentation électrique pour une tension (24) de polarisation sur la partie (22) de superposition de tension de polarisation et une borne de commande qui commande le commutateur (25) depuis un extérieur pour être activé ou désactivé.

5. Appareil (21D) de génération de tension de polarisation selon la revendication 1, dans lequel
le circuit d'alimentation électrique pour une tension (24) de polarisation a la batterie secondaire (24a) et un convertisseur CC/CC (24b) qui accroît une tension de CC de la batterie secondaire (24a) et produit la tension de polarisation.

6. Appareil (21D) de génération de tension de polarisation selon la revendication 2, dans lequel
l'appareil (21D) de génération de tension de polarisation est placé dans un connecteur dans la sonde (2D, 2E), le connecteur étant connecté de manière détachable à l'appareil (3A, 3B) d'observation par ultrasons.

7. Appareil (21D) de génération de tension de polarisation selon la revendication 2, dans lequel
l'appareil (21D) de génération de tension de polarisation a une pluralité de premières bornes de connecteur qui sont connectées de manière détachable à une pluralité de bornes de récepteur de connecteur d'un récepteur de connecteur de l'appareil (3A, 3B) d'observation par ultrasons et a une pluralité de deuxièmes bornes de connecteur qui sont connectées de manière détachable à des bornes de récepteur de connecteur d'un récepteur de connecteur d'une chargeur (4) générant une tension de charge pour charger la batterie secondaire (24a).

8. Appareil (21D) de génération de tension de polarisation selon la revendication 4, dans lequel
l'appareil (21D) de génération de tension de polarisation a en outre un chargeur (4) qui est connecté de manière détachable à l'appareil (21D) de génération de tension de polarisation et génère une tension de charge pour charger la batterie secondaire (24a), et le chargeur (4) a un circuit (33) de commande de commutateur qui désactive le commutateur par l'intermédiaire de la borne de commande si le chargeur (4) est connecté à l'appareil (21D) de génération de tension de polarisation.

9. Système (1, 1B) de diagnostic par ultrasons comprenant :
une sonde (2D, 2E) à ultrasons qui est équipée avec un transducteur (11A) capacitif ;
un appareil (3A, 3B) d'observation par ultrasons qui incorpore un circuit (41) de transmission générant un signal de transmission pour faire en sorte que le transducteur (11A) capacitif génère des ultrasons, un circuit (42) de réception exécutant un traitement sur un signal reçu délivré en sortie du transducteur (11A) capacitif à la réception d'ultrasons, et un circuit (43) de séparation de transmission/réception qui sépare le signal de transmission du signal reçu ; et
un appareil (21D) de génération de tension de polarisation qui est incorporé dans ou connecté de manière détachable à la sonde (2D, 2E) à ultrasons et inclut un circuit d'alimentation électrique pour une tension (24) de polarisation incluant une batterie secondaire (24a) rechargeable pour produire une tension de polarisation destinée à être appliquée au transducteur (11A) capacitif et produire la tension de polarisation et une partie (22) de superposition de tension de polarisation superposant la tension de polarisation sur le signal de transmission délivré en sortie jusqu'à l'extérieur de l'appareil (3A, 3B) d'observation par ultrasons et sur le signal reçu destiné à être entré à l'intérieur de l'appareil (3A, 3B) d'observation par ultrasons,
**caractérisé par**
une partie (26) de détection de niveau de charge qui détecte un niveau de charge de la batterie secondaire (24a) constituant le circuit d'alimentation électrique pour une tension (24) de polarisation ;
une partie (72) de notification de niveau de charge qui notifie une information de niveau de charge détecté par la partie (26) de détection de niveau de charge ; et
un amplificateur (71) à gain variable qui fait varier un gain pour amplifier le signal reçu destiné à être entré à l'intérieur de l'appareil d'observation de diagnostic par ultrasons sur la base du niveau de charge détecté par la partie (26) de détection de niveau de charge, dans lequel
lorsque la tension de polarisation est devenue inférieure à une valeur limite inférieure d'une plage appropriée de tension de polarisation, la partie (26) de détection de niveau de charge compense une chute de sensibilité au signal reçu causée lorsque la tension de polarisation chute en-dessous de la plage appropriée par l'intermédiaire d'une augmentation du gain de l'amplificateur (71) à gain variable.

10. Système (1, 1B) de diagnostic par ultrasons selon la revendication 9, dans lequel
l'appareil (21D) de génération de tension de polarisation comprend en outre un commutateur (25) qui active ou désactive la superposition de la tension de polarisation générée par le circuit d'alimentation électrique pour une tension (24) de polarisation sur la partie (22) de superposition de tension de polarisation et une borne de commande qui commande le commutateur (25) d'un extérieur de l'appareil (21D) de génération de tension de polarisation pour être activé ou désactivé.

11. Système (1, 1B) de diagnostic par ultrasons selon la revendication 10, dans lequel
le système (1, 1B) a en outre un deuxième appareil (3B) d'observation qui incorpore un circuit (47B) de commande de commutateur générant, pour le commutateur (25), un signal de commande de commutateur pour désactiver la superposition de la tension de polarisation, un deuxième circuit (41B) de transmission générant un deuxième signal de transmission pour faire en sorte que le transducteur (11A) capacitif génère des ultrasons, un deuxième circuit (42B) de réception exécutant un traitement de signal sur un signal reçu délivré en sortie du transducteur (11A) capacitif, et un circuit de tension de polarisation générant une deuxième tension de polarisation destinée à être superposée sur un signal de transmission provenant du deuxième circuit (41B) de transmission et le signal reçu entré dans le deuxième circuit (42B) de réception et superposant la deuxième tension de polarisation, en plus de l'appareil d'observation par ultrasons, dans lequel un signal reçu avec la tension de polarisation superposée générée par l'appareil (21D) de génération de tension de polarisation est entré.

12. Système (1, 1B) de diagnostic par ultrasons selon la revendication 9, dans lequel
le circuit d'alimentation électrique pour une tension (24) de polarisation a la batterie secondaire (24a) et un convertisseur CC/CC (24b) qui accroît une tension de CC de la batterie secondaire (24a) et produit la tension de polarisation.

13. Système (1, 1B) de diagnostic par ultrasons selon la revendication 10, dans lequel
le système (1, 1B) a en outre un chargeur (4) qui est connecté de manière détachable à l'appareil (21D) de génération de tension de polarisation et génère une tension de charge pour charger la batterie secondaire (24a), et le chargeur (4) a un circuit (33) de commande de commutateur qui désactive le commutateur par l'intermédiaire de la borne de commande si le chargeur (4) est connecté à l'appareil (21D) de génération de tension de polarisation.

14. Système (1, 1B) de diagnostic par ultrasons selon la revendication 10, dans lequel
la sonde (2D, 2E) à ultrasons est composée d'une sonde à ultrasons à base de balayage mécanique qui exécute mécaniquement un balayage aux ultrasons en utilisant le transducteur (11A) capacitif.

15. Système (1, 1B) de diagnostic par ultrasons selon la revendication 10, dans lequel
la sonde (2D, 2E) à ultrasons a une matrice d'une pluralité de transducteurs capacitifs comme le transducteur capacitif et est composée d'une sonde à ultrasons à base de balayage électronique qui exécute électroniquement un balayage aux ultrasons en pilotant la matrice des transducteurs capacitifs.
